# FASCICULE DE BREVET EUROPEEN

(11) **EP 0 847 747 B1**
(45) Date de publication et mention de la délivrance du brevet: **15.01.2003**
(21) Numéro de dépôt: 97402753.4
(22) Date de dépôt: 17.11.1997
(51) Int. Cl.: A61K 7/00, A61K 7/13, C09B 63/00, C09B 67/00

(54) **Pigment mélanique composite sous forme de particules comprenant un noyau sphérique à base de cire, procédé de préparation et utilisations en cosmétique**
Verbundteilchen von Melaninpigment mit einem kugelförmigen Wachskern, Herstellungsverfahren und Verwendungen in der Kosmetik
Composite melanin pigment particles having a wax spherical core, preparation process and uses in cosmetics

(30) Priorité: 16.12.1996 FR 9615450
(43) Date de publication de la demande: 17.06.1998
(73) Titulaire: L'OREAL, 75008 Paris (FR)
(72) Inventeur: Nicolas-Morgantini, Luc, 60810 Rully (FR); Lety, Alain, 77400 Lagny sur Marne (FR); Vanlerberghe, Guy, 77410 Villeparisis (FR)
(74) Mandataire: Dodin, Catherine

(56) Documents cités:
- EP-A- 0 441 689
- EP-A- 0 518 773
- WO-A-93/12761
- WO-A-93/13743
- WO-A-93/13744
- WO-A-93/13745
- WO-A-93/19721
- WO-A-94/25531

## Description

La présente invention a trait à un pigment mélanique composite sous forme de particules constituées d'un noyau sphérique à base de cire et d'une couche externe enveloppant ledit noyau comprenant au moins un composé résultant de la polymérisation oxydative d'un précurseur de pigment de type mélanique ainsi que ses utilisations sous forme de poudre ou sous forme de dispersion aqueuse, dans des compositions cosmétiques notamment des produits de coloration et/ou de maquillage et/ou de soin des matières kératiniques.

On utilise généralement dans les compositions de maquillage pour la peau et pour les phanères, des pigments à base de composés métalliques tels que par exemple des oxydes de fer noir et brun.
On recherche depuis quelques années des nouveaux systèmes de pigment pouvant également être utilisés dans des compositions de teinture capillaire conférant aux cheveux une coloration qui puisse être rapidement éliminée.
On connaît dans la demande de brevet français FR2686345 des pigments composites constitués de particules minérales colorées de dimension inférieure à 200 µm comportant en leur sein et/ou sur leur surface un pigment mélanique synthétique formé in situ par oxydation d'un composé indolique.
On connaît également dans la demande de brevet français FR2686248 des pigments composites constitués de particules minérales ou organiques de dimension inférieure à 200 µm comportant en leur sein et/ou sur leur surface un pigment mélanique synthétique formé in situ par oxydation d'un composé indolinique.
On connaît encore, par WO93/12761, des particules solides complexes comprenant un produit biologiquement actif régulièrement réparti à la surface d'une substance support ou grain.

La Demanderesse a découvert de manière surprenante de nouveaux pigments composites mélaniques sous forme de microparticules de cire enrobées d'un pigment synthétique formé in situ par oxydation d'un précurseur de pigment mélanique qui, de par la taille sensiblement réduite des particules qui les constituent, se répartissent mieux et de manière plus homogène dans les formulations cosmétiques, permettent un meilleur étalement de la composition sur la peau ou les phanères et présentent un pouvoir couvrant plus important.

La Demanderesse a constaté de manière inattendue que les pigments mélaniques composites de l'invention présentaient en outre des propriétés filmogènes satisfaisantes.
De plus, les pigments composites de l'invention sont stables en dispersion aqueuse. Ils présentent également, par leur constitution et leur très faible taille de particule, l'avantage de présenter une fraction volumique apparente du colorant formé in situ par oxydation du précurseur de pigment mélanique plus importante. Cette propriété a pour conséquence de pouvoir introduire dans les particules composites de l'invention des quantités plus faibles de précurseur de pigment mélanique pour obtenir un même effet colorant.

Les pigments composites mélaniques de l'invention présentent une grande variété de nuances et peuvent être utilisés dans de nombreux types de produits de maquillage.

Les pigments composites mélaniques de l'invention présentent en outre un coefficient d'absorption des radiations ultraviolettes satisfaisant pour être utilisés dans des produits protégeant l'épiderme humain des effets des rayonnements UV.

D'autre part, les pigments composites mélaniques de l'invention agissent efficacement contre les radicaux libres oxygénés en particulier contre les radicaux libres hydroxyle (°OH) hautement nocifs et permettent notamment de lutter contre le vieillissement cutané et de protéger la peau et les phanères contre les effets des radicaux libres induits par exemple par les polluants atmosphériques et/ou par le rayonnement ultraviolet.

On appellera par la suite « phanères » : les cheveux, les poils tels que les cils et les sourcils ou les ongles.

L'invention concerne donc des pigments mélaniques composites sous forme de particules constituées d'un noyau sphérique de diamètre inférieur à 1 µm comprenant au moins une cire et au moins un tensioactif émulsionnant et d'une couche externe enveloppant ledit noyau comprenant au moins un composé résultant de la polymérisation oxydative d'un précurseur de pigment de type mélanique.

Les particules de cire ont de préférence des dimensions inférieures à 500 nm. Elles sont généralement essentiellement constituées de cire ou d'un mélange de cires dont le point de fusion varie de 50 à 100°C. Elles contiennent en plus un ou plusieurs tensioactifs émulsionnants et éventuellement un ou plusieurs additifs gras huileux ou pâteux et/ou un ou plusieurs actifs liposolubles que l'on précisera plus loin.

La cire ou le mélange de cires contenus dans le noyau des particules du pigment composite de l'invention, représente en général de 20 à 97% en poids et plus préférentiellement de 40 à 85 % du poids total du noyau.
Les cires ou les mélanges de cires utilisés sont choisis de préférence parmi la cire de Carnauba, la cire de Candellila, la cire d'Alfa et leurs mélanges. En plus des cires ou des mélanges de cires cités ci-dessus, on peut également utiliser:
- la cire de paraffine
- l'ozokérite
- les cires végétales comme la cire d'olivier, la cire de riz, la cire de jojoba hydrogénée, les cires absolues de fleurs comme la cire essentielle de fleur de cassis ;
- les cires animales comme les cires d'abeille, les cires d'abeille modifiées ;
- les cires marines telles que celles vendues sous le nom M82 par SOPHIM
- les céramides naturelles ou synthétiques
- les cires de polyéthylène.
De façon préférentielle, le noyau des particules de pigment composite contient au moins 20 % en poids et plus préférentiellement au moins 50% en poids de cire choisie parmi la cire de Carnauba, la cire de Candellila, la cire d'Alfa et leurs mélanges par rapport à la quantité totale de cire présente dans ledit noyau.

En plus des cires ou des mélanges de cires cités ci-dessus, on peut également utiliser un ou plusieurs additifs gras huileux ou pâteux tels que par exemple :
- les huiles végétales comme l'huile de tournesol ou l'huile de jojoba,
- les huiles minérales comme l'huile de paraffine,
- les huiles siliconées fluides,
- les huiles et cires fluorées,
- la vaseline,
- la lanoline.
Les huiles et/ou les additifs gras additionnels dans le noyau des particules de pigment composite de l'invention peuvent représenter jusqu'à 30% en poids du poids total du noyau.

En plus des cires ou des mélanges de cires cités ci-dessus, on peut également utiliser un ou plusieurs actifs liposolubles tels que des filtres UV liposolubles, des vitamines liposolubles. Ils peuvent représenter jusqu'à 30% en poids et plus préférentiellement jusqu'à 10% en poids du poids total du noyau.

Le ou les tensioactifs émulsionnants présents dans le noyau cireux des particules de pigment composite de l'invention sont choisis parmi les tensioactifs anioniques, canoniques, non-ioniques et leurs mélanges, habituellement utilisés dans la préparation des microdispersions de cire en particulier les tensioactifs utilisés dans les microdispersions décrites dans la demande de brevet EP-A-0557 196. Ils sont présents dans des concentrations allant de préférence de 3 à 50% en poids et plus particulièrement de 10 à 30% en poids par rapport au poids total du noyau cireux.
Le rapport pondéral cire(s)/tensioactif(s) varie de préférence de 1 à 30 et plus particulièrement de 2 à 10.

Selon une forme particulièrement préférée de l'invention, le noyau des particules de pigment composite de l'invention comporte en plus un composé amphiphile non-ionique à longue tête polaire comme agent stabilisant des particules de cire constituant le noyau des pigments composites.

En effet, la Demanderesse a constaté que les dispersions aqueuses de pigments composites et de particules de cire tels que définis ci-dessus, en présence de certains additifs tels que des sels, des conservateurs ou des parfums pouvaient se déstabiliser en raison d'un phénomène de floculation des particules de cire et des particules de pigment constituant leur noyau.
La Demanderesse a découvert de façon surprenante que l'utilisation de molécules amphiphiles non-ioniques à longue tête polaire permet d'éviter ces problèmes de floculation des particules de cire et des particules de pigment en suspension aqueuse.

Les molécules amphiphiles non-ioniques stabilisantes de l'invention ont en général d'une part une chaîne hydrocarbonée pouvant être linéaire ou ramifiée, saturée ou insaturée, aliphatique ou aromatique et d'autre part comportent au moins 50 moles de motifs polaires ou hydrophiles constituant la tête polaire. Ces motifs polaires peuvent être par exemple des motifs oxyde d'éthylène, oxyde de propylène, glycérol ou acrylamide.

Elles sont choisies notamment parmi les acides gras ou les amides polyalcoxylés et/ou polyglycérolés, les esters d'acide gras et de polyols polyalcoxylés et/ou polyglycérolés, les alcools gras ou les alkylphénois polyalcoxylés et/ou polyglycérolés, les alcanediols ou alcènediols-1,2 ou 1,3 polyalcoxylés et/ou polyglycérolés, les thioéthers de polyacrylamide et de dodécyle, les dérivés alcoxylés des acides gras ou des alcool gras de la lanoline comportant au moins 50 moles de motifs polaires ou hydrophiles constituant la tête polaire.

On choisit plus particulièrement les composés amphiphiles non-ioniques à longue tête polaire de formule suivante :

RO-(CH₂CH₂O)ₙ-H

dans laquelle R désigne un radical alkyle, alcényle ou aryle comportant au moins 8 atomes de carbone et n est un nombre allant de 70 à 200 et plus particulièrement de 100 à 200. Ces molécules sont décrites et préparées dans la demande de brevet japonais JP-A-6106581 1.

Les composés amphiphiles non-ioniques à longue tête polaire sont présents dans des concentrations allant de préférence de 1 à 40% en poids et plus particulièrement de 5 à 20% en poids par rapport au poids total du noyau cireux. Le rapport pondéral tensioactif(s) émulsionnant(s)/ composé(s) amphiphile(s) non-ionique(s) varie de préférence de 1 à 50 et plus particulièrement de 1 à 20.

Les précurseurs de pigment mélanique qui sont utilisés conformément à l'invention et qui vont conduire par polymérisation oxydative à la formation in situ d'un pigment synthétique autour des microparticules de cire, sont choisis de préférence parmi :
(i) les composés indoliniques de formule suivante et leurs sels d'addition avec un acide : 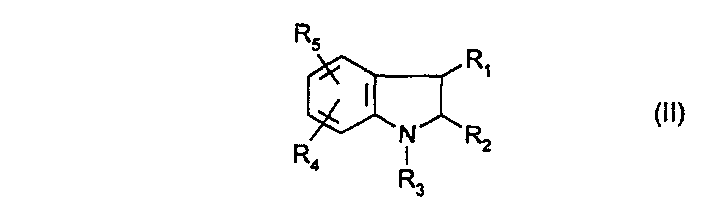 dans laquelle:
   R1 et R3 représentent, indépendamment l'un de l'autre, un atome d'hydrogène ou un groupement alkyle en C1-C4;
   R2 désigne un atome d'hydrogène, un groupement alkyle en C1-C4, un groupement carboxyle, ou alcoxy (C1-C4) carbonyle;
   R4 désigne un atome d'hydrogène, un groupement alkyle en C1-C4, un groupement hydroxyle, un groupement alcoxy (C1-C4) amino, un groupement alkyl (C1-C10) amino ou un atome d'halogène ;
   R5 désigne un atome d'hydrogène, un groupement alkyle en C1-C4, un groupement hydroxyle, un groupement alcoxy en C1-C4 ou amino ;
   au moins l'un des radicaux R4 ou R5 désignant un groupe hydroxyle, alcoxy, ou amino; sous réserve que lorsque R5 désigne amino alors R4 est différent d'un radical alkylamino ;
   R4 et R5 peuvent former un cycle alkylène dioxy et sont en position 5 et 6.
(ii) les composés indoliques de formule suivante et leurs sels d'addition avec un acide : 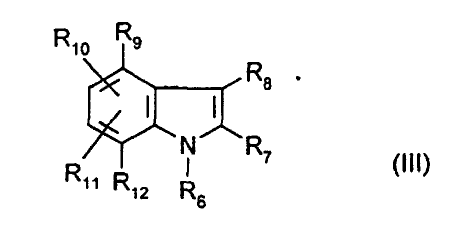 dans laquelle :
   R6 et R8 représentent, indépendamment l'un de l'autre, un atome d'hydrogène ou un groupement alkyle en C1-C4;
   R7 désigne un atome d'hydrogène, un groupement alkyle en C1-C4, un groupement carboxyle, ou alcoxy (C1-C4) carbonyle ;
   R9 et R12 désignent, indépendamment l'un de l'autre, un atome d'hydrogène, un groupement alkyle en C1-C4, un groupement hydroxyle, un groupement alcoxy en C1-C4, amino, un groupement acyl (C2-C4)oxy, acyl (C2-C4)amino ;
   R10 désigne un atome d'hydrogène, un groupement alkyle en C1-C4, un groupement hydroxyle, un groupement alcoxy en C1-C4, amino, un groupement acyl (C2-C4)oxy, acyl (C2-C4)amino, un atome d'halogène ou un groupe triméthylsilyloxy;
   R11 désigne un atome d'hydrogène, un groupement hydroxyle, un groupement alcoxy en C1-C4, amino, un groupement acyl(C2-C4)oxy, acyl(C2-C4)amino, hydroxyalkyl(C2-C4)amino ou un groupe triméthylsilyloxy ;
   R10 et R11 peuvent former, conjointement avec les atomes de carbone auxquels ils sont attachés, un cycle méthylènedioxy éventuellement substitué par un alkyle en C1-C4, un alcoxy en C1-C4 ou un cycle carbonyldioxy;
   au moins l'un des groupements R9 à R12 représente un groupe OZ ou NHR et un seul desdits groupements désigne NHR;
   au plus deux des groupements R9 à R12 représente un groupe OZ, lorsque Z désigne hydrogène et sont en position 5 et 6;
   au moins l'un des groupements R9 à R12 désigne hydrogène, dans le cas où un seul de ces groupements désignent hydrogène, un seul desdits groupement désigne un groupe NHR ou OZ et les autres désignent un groupe alkyle en C1-C4;
   R désigne un atome d'hydrogène, un groupe acyle en C2-C4, un groupe hydroxyalkyle en C2-C4 ;
   Z désigne un atome d'hydrogène, un groupe acyle en C2-C14, un groupe alkyle en C1-C4, triméthylsilyle.
(iii) leurs mélanges.

Les sels des composés indoliques ou indoliniques de formule (Il) ou (III) précités sont des sels cosmétiquement acceptables choisis par exemple parmi les chlorhydrates, bromohydrates, sulfates, méthanesulfonates.

Les composés de formule (II) et leurs sels correspondants sont décrits dans la demande de brevet français FR2686248 et les composés de formule (III) et leurs sels correspondants sont décrits dans la demande de brevet français FR2686345.

Parmi les composés de formule (Il) et leurs sels, on peut citer plus particulièrement la 5,6-dihydroxyindoline et ses sels d'addition avec un acide comme les bromhydrates.
Parmi les composés de formule (III) et leurs sels, on peut citer plus particulièrement le 6-hydroxyindole, le 5,6-dihydroxyindole et le N-méthyl 5,6-dihydroxyindole et leurs sels d'addition avec un acide.

Le ou les précurseurs de pigment mélanique est ou sont utilisés de préférence dans des quantités telles que le rapport pondéral cire(s)/colorant formé in situ après oxydation soit supérieur ou égal à 3.

La taille des particules sphériques de pigment mélanique composite selon l'invention est de préférence inférieure à 1 µm et plus préférentiellement inférieure ou égale à 500 nm et plus particulièrement varie de 20 à 500 nm.

Un autre objet de la présente invention est un procédé de préparation des pigments mélaniques composites tels que définis-précédemment.
Ce procédé consiste essentiellement à disperser et à solubiliser, à température ambiante, sous agitation douce et sous atmosphère inerte, un précurseur de type mélanique tel qu'indiqué ci-dessus dans une dispersion aqueuse de particules sphériques de cire de dimension inférieure à 1 µm contenant en plus de la cire au moins un tensioactif émulsionnant, telles que celles définies ci-dessus puis à oxyder ledit précurseur de manière à le polymériser et à former une couche externe enveloppant chaque particule de cire.

L'étape de polymérisation s'effectue en général en dessous de la température de fusion de la cire ou du mélange de cire constituant les particules de cire et de préférence à température ambiante, pour éviter la fusion des particules de cire. Elle peut être réalisée en milieux aqueux par auto-oxydation à l'air provoquée par une agitation vigoureuse de la microdispersion de cire ajustée à un pH neutre ou alcalin.

Selon un autre type de procédé, la préparation des produits conformes à l'invention peut s'effectuer en présence d'un agent oxydant tel que le peroxyde d'hydrogène, les peracides et les persels.
On peut citer parmi les peracides et les persels, l'acide périodique et ses sels hydrosolubles, les permanganates et les bichromates, tels que de sodium ou de potassium, le persulfate d'ammonium et les peracides organiques. Le sel d'acide périodique préféré est le periodate de sodium.
D'autres agents oxydants sont choisis parmi les chlorites alcalins, l'oxyde d'argent, le chlorure ferrique, l'oxyde de plomb, le nitrite de sodium ; les sels de terres rares tels que notamment le sel de cérium.
On peut également utiliser des oxydants organiques choisis parmi les ortho et parabenzoquinones, les ortho et parabenzoquinones monoimines et diimines, les 1,2- et 1,4-naphtoquinones, les 1,2 et les 1,4-naphtoquinones mono- ou diimines.
L'oxydation peut enfin être effectuée par utilisation d'iodure tel qu'un iodure de métal alcalin, alcalino-terreux ou d'ammonium en présence de peroxyde d'hydrogène.

Ces agents oxydants peuvent être activés éventuellement par un agent modificateur de pH. Les agents modificateurs de pH sont des agents acidifiants ou alcalinisants habituellement utilisés en cosmétique.
Il est également possible de procéder par oxydation par voie enzymatique. Cette oxydation s'effectue dans un milieu oxydant et en présence d'une enzyme à activité oxydante ou peroxydante telle que les enzymes choisies parmi la peroxydase de raifort, la chloroperoxydase, la peroxydase du lait, la cytochrome C-peroxydase, ainsi que des produits ayant une activité similaire, celle des enzymes peroxydantes telle que l'hémoglobine, la methémoglobine, la myoglobine, la metmyoglobine.
On utilise de préférence comme agents oxydants, le peroxyde d'hydrogène, l'acide périodique et ses sels, le permanganate de potassium, l'hypochlorite de sodium, le persulfate d'ammonium, le nitrite de sodium et le système iodure/peroxyde d'hydrogène. Lorsqu'on utilise un iodure en présence d'eau oxygénée, il s'agit préférentiellement d'iodure de sodium ou de potassium à une concentration pondérale comprise entre 1 et 6% par rapport au poids du milieu réactionnel.

Selon un mode préféré de mise en oeuvre du procédé de l'invention, on incorpore à chaud ou à froid, un composé amphiphile non-ionique à longue tête polaire tel que ceux précités dans la microdispersion de cire avant de disperser dans celle-ci le précurseur de pigment mélanique. Ce composé amphiphile permet de stabiliser la microdispersion de cire et d'éviter les problèmes de floculation évoqués plus haut.

A l'issue de la polymérisation on obtient une dispersion aqueuse de particules de pigment composite conformes à la présente invention, opaque, fluide et stable au cours du temps et dont la couleur varie selon le choix du précurseur mélanique, dans une large gamme de teintes.

Cette dispersion peut être déshydratée selon les techniques de lyophilisation ou d'atomisation de manière à obtenir les particules de pigment composite sous forme pulvérulente tout en conservant leur granulométrie qu'elles avaient en suspension.

Un autre objet de l'invention consiste en une composition cosmétique ou dermatologique comprenant au moins un pigment mélanique composite sous forme de particules sphériques à l'état de poudre ou en dispersion aqueuse telles que définies précédemment.

Les pigments mélaniques composites conformes à l'invention, dans leur application cosmétique ou dermatologique, sont utilisés sous forme de poudre ou bien en dispersion aqueuse dans des compositions cosmétiques, à une concentration variant de préférence de 0,1 à 35% en poids et en particulier de 0,5 à 20 % en poids par rapport au poids total de la composition.

Ils peuvent être utilisés dans et pour la préparation de produits de maquillage, notamment des cils, des sourcils, de la peau, des ongles, tels que sous forme de fard à paupières, fards à joues, fonds de teint; ligneurs encore appelés "eyeliners" ou mascaras pour les cils et les sourcils ; les vernis à ongles.
Ils peuvent être aussi utilisés dans et pour la préparation de produits de compositions tinctoriales pour cheveux, notamment pour réaliser une teinture temporaire ou de maquillage des cheveux.
Ils peuvent être utilisés dans et pour la préparation de compositions protectrices de l'épiderme humain et/ou des phanères contre les effets néfastes des rayons UV en particulier de produits dits solaires.
Ils peuvent être utilisés dans et pour la préparation de compositions protectrices de la peau et/ou des phanères contre les effets des radicaux libres induits par exemple par les polluants atmosphériques et/ou par le rayonnement ultra-violet.

Lorsque les compositions cosmétiques ou dermatologiques sont des produits de maquillage de la peau, des cils et des sourcils, elles peuvent notamment se présenter sous forme solide ou pâteuse, comme des émulsions huile-dans-eau ou eau-dans-huile ou encore des suspensions. Les compositions peuvent se présenter notamment sous forme de gel, de crème, de lait, de poudre, de stick ou de mousse aérosol.
Lorsque les compositions cosmétiques ou dermatologiques sont utilisées pour la protection de l'épiderme humain et/ou des phanères contre les rayonnements UV, elles peuvent se présenter sous forme de suspensions ou de dispersions ou encore sous forme d'émulsions telles que crèmes et laits, de pommades, de gels, de bâtonnets solides ou de mousses aérosols. Lorsqu'elles sont utilisées sous forme d'émulsions, elles peuvent contenir en outre des agents tensioactifs bien connus dans l'état de la technique, tels que des agents tensioactifs anioniques, non ioniques, canoniques ou amphotères.

Les compositions cosmétiques ou dermatologiques de l'invention peuvent également contenir des corps gras, des solvants organiques, des silicones, des épaississants, des adoucissants, des filtres solaires, des agents anti-mousse, des agents hydratants, des parfums, des conservateurs, des agents anti-oxydants, des charges, des séquestrants, des agents de traitement tels que des polymères anioniques, canoniques, non ioniques, amphotères, ou leurs mélanges, des propulseurs, des agents alcalinisants ou acidifiants.

Les compositions cosmétiques ou dermatologiques conformes à l'invention peuvent également contenir en plus des pigments composites mélaniques tels que définis ci-dessus, des pigments généralement utilisés en cosmétique, notamment des pigments nacrés et/ou nacrants permettant de varier les colorations susceptibles d'être obtenues, ou d'augmenter la protection vis-à-vis du rayonnement ultraviolet. On utilise, dans ce dernier cas, plus particulièrement des pigments ou nanopigments d'oxydes métalliques tels que les oxydes de titane, de zinc, de cérium ou de zirconium. Les nanopigments qui sont utilisés de façon préférentielle, sont des pigments ayant un diamètre moyen inférieur à 100 nm et de préférence compris entre 5 et 50 nm. Ils peuvent être enrobés ou non enrobés.
Les pigments enrobés sont des pigments qui ont subi un ou plusieurs traitements de surface de nature chimique, électronique, mécanochimique et/ou mécanique avec des composés tels que décrits par exemple dans COSMETICS and TOILETRIES, Février 1990, Vol. 105 pages 53-64, tels que des aminoacides, de la cire d'abeille, des acides gras, des alcools gras, des tensioactifs anioniques, des lécithines, des sels de sodium, potassium, zinc, fer ou aluminium d'acides gras, des alcoxydes métalliques (de titane ou d'aluminium), du polyéthylène, des silicones, des protéines (collagène, élastine), des alcanolamines, des oxydes de silicium, des oxydes métalliques ou de l'hexamétaphosphate de sodium.

Lorsque les compositions cosmétiques de l'invention sont utilisées pour le traitement des ongles, le pigment composite mélanique de l'invention est introduit dans un milieu pour vernis à ongles comprenant un solvant volatil et des polymères. Ces produits peuvent être appliqués directement sous forme de poudre ou au moyen de compositions cosmétiques telles que définies ci-dessus.

Les exemples suivants sont destinés à illustrer l'invention.

### Exemples de préparation de microdispersions de pigments mélaniques composites

### EXEMPLE A

- Cire de Carnauba 10% en poids
- Alcool de lanoline et mélange d'alcools gras polyoxyéthylénés (25 OE) vendu sous le nom SOLULAN 25 par la société AMERCHOL (tensioactif émulsionnant) 2,5% en poids
- Alcool stéarylique polyoxyéthyléné vendu sous le nom BRIJ 700 par la société ICI (agent stabilisant) 2% en poids
- Produit résultant de l'oxydation du 5,6-dihydroxyindole 1% en poids
- Tampon KH2PO,,/K2HP04 0,5M pH7 10% en poids
- Eau qsp 100%

### Etape 1 : Préparation de la microdispersion de cire

Dans un flacon de verre blanc de 250 ml à large ouverture, on introduit les ingrédients suivants
- Cire de Camauba 17,235g
- SOLULAN 25 (tensioactif émulsionnant) 4,305g

On porte à 90°C la cire et le tensioactif émulsionnant en homogénéisant sous agitation modérée. En continuant d'agiter, on incorpore 128,46 g d'eau portée également à 90°C.
La microémulsion obtenue est ramenée à température ambiante et forme une microdispersion de grains à base de cire.
Diamètre moyen des particules de cire : 112 nm
Polydispersité en taille des particules : 0,06 (mesurée par diffusion quasi-élastique de la lumière, à l'aide d'un appareil du type AMTEC/BI 2030 AT)

### Etape 2: Stabilisation de la microdispersion de cire

Dans un flacon de verre blanc de 125 ml à large ouverture, on introduit 2,475 g d'agent stabilisant (BRIJ 700) et on complète à 110 g par la microdispersion de cire obtenue dans l'étape 1, refroidie. On solubilise l'agent stabilisant dans la dite dispersion sous agitation magnétique.
La microdispersion de cire obtenue a pour granulométrie :
Diamètre moyen des particules de cire : 119 nm
Polydispersité en taille des particules : 0,07 (mesurée par diffusion quasi-élastique de la lumière, à l'aide d'un appareil du type AMTEC/BI 2030 AT)

### Etape 3 : Préparation de la microdispersion de pigment composite mélanique

### - Solubilisation du précurseur mélanique dans la microdispersion de cire

Dans un flacon de verre blanc de 125 ml à large ouverture, on introduit 1,11 g de 5,6-dihydroxyindole puis on complète à 100 g avec la microdispersion obtenue dans l'étape 2.
On introduit de l'azote dans ledit flacon au dessus du mélange obtenu, ainsi qu'un barreau magnétique et on referme aussitôt le flacon. On solubilise le 5,6-dihydroxyindole sous agitation magnétique pendant 2 heures.

### - Polymérisation in situ du précurseur mélanique dans la microdispersion de cire

On introduit dans un autre flacon de verre blanc de 125 ml à large ouverture, 10g de solution tampon KH2PO4/K2HP04 0,5M et de pH7 puis on complète à 100g avec la microdispersion de cire contenant le 5,6-dihydroxyindole. On introduit un barreau magnétique puis on ferme ledit flacon avec une capsule perforée.
On laisse agir le mélange sous agitation magnétique pendant 48 heures jusqu'à la fin de la polymérisation du 5,6-dihydroxyindole.

On obtient une dispersion de particules de pigment composite d'aspect liquide, brun-noir opaque et présentant la granulométrie suivante :
Diamètre moyen des particules de cire : 122 nm
Polydispersité en taille des particules : 0,08 (mesurée par diffusion quasi-élastique de la lumière, à l'aide d'un appareil du type AMTEC/BI 2030 AT)

### EXEMPLE B

- Cire de Camauba 9% en poids
- N-oléoyidihydrosphingosine (céramide) 1% en poids
- Alcool de lanoline et mélange d'alcools gras polyoxyéthylénés (25 OE) vendu sous le nom SOLULAN 25 par la société AMERCHOL (tensioactif émulsionnant) 2,5% en poids
- Alcool stéarylique polyoxyéthyléné vendu sous le nom BRIJ 700 par la société ICI (agent stabilisant) 2%en poids
- Produit résultant de l'oxydation du 5,6-dihydroxyindole 1% en poids
- Tampon KH2PO4/K2HP04 0,5M pH7 10% en poids
- Eau qsp 100% en poids

On procède dans les mêmes conditions des étapes 1 à 3 du procédé de préparation de l'exemple A avec les ingrédients suivants
- Cire de Carnauba 15,51 g
- N-oléoyldihydrosphingosine (céramide) 1,725 g
- SOLULAN 25 4,305 g

La N-oléoyldihydrosphingosine est utilisée avec la cire dans les mêmes conditions indiquées dans l'étape 1 du procédé de l'exemple A.

La microdispersion de cire obtenue dans l'étape 1 a pour granulométrie
Diamètre moyen des particules de cire : 108 nm
Polydispersité en taille des particules : 0,09 (mesurée par diffusion quasi-élastique de la lumière, à l'aide d'un appareil du type AMTEC/BI 2030 AT)

La microdispersion de cire obtenue dans l'étape 2 a pour granulométrie :
Diamètre moyen des particules de cire : 116 nm
Polydispersité en taille des particules : 0,1 (mesurée par diffusion quasi-élastique de la lumière, à l'aide d'un appareil du type AMTEC/BI 2030 AT)

On obtient dans l'étape 3 une dispersion de particules de pigment mélanique composite d'aspect liquide, brun-noir opaque et présentant la granulométrie suivante :
Diamètre moyen des particules de cire : 124 nm
Polydispersité en taille des particules : 0,09 (mesurée par diffusion quasi-élastique de la lumière, à l'aide d'un appareil du type AMTEC/BI 2030 AT)

### EXEMPLE C

- Cire de Camauba 9% en poids
- 1-(2'-F-hexyléthylthio)-3-(2"-éthyl hexyloxy)-propane2-ol (huile fluorée) 1 % en poids
- Alcool de lanoline et mélange d'alcools gras polyoxyéthylénés (25 OE) vendu sous le nom SOLULAN 25 par la société AMERCHOL 2,5% en poids
- Alcool stéarylique polyoxyéthyléné vendu sous le nom BRIJ 700 par la société ICI 2% en poids
- Produit résultant de l'oxydation du 5,6-dihydroxyindole 1 % en poids
- Tampon KH2PO,,/K2HP04 0,5M pH7 10% en poids
- Eau 74,5% en poids

On procède dans les conditions des étapes 1 à 3 du procédé de préparation de l'exemple A avec les ingrédients suivants :
- Cire de Camauba 15,510 g
- 1-(2'-F-hexyléthylthio)-3-(2"-éthyl hexyloxy)-propane 2-ol (huile fluorée) 1,725 g
- SOLULAN 25 4,305 g

L'huile fluorée est utilisée avec la cire dans les mêmes conditions indiquées dans l'étape 1 du procédé de l'exemple A.

La microdispersion de cire obtenue dans l'étape 1 a pour granulométrie
Diamètre moyen des particules de cire 108 nm
Polydispersité en taille des particules 0,07 (mesurée par diffusion quasi-élastique de la lumière, à l'aide d'un appareil du type AMTEC/BI 2030 AT)

La microdispersion de cire obtenue dans l'étape 2 a pour granulométrie
Diamètre moyen des particules de cire 114 nm
Polydispersité en taille des particules 0,07 (mesurée par diffusion quasi-élastique de la lumière, à l'aide d'un appareil du type AMTEC/BI 2030 AT)

On obtient dans l'étape 3 une dispersion de particules de pigment mélanique composite d'aspect liquide, brun-noir opaque et présentant la granulométrie suivante :
Diamètre moyen des particules de cire 116 nm
Polydispersité en taille des particules 0,08 (mesurée par diffusion quasi-élastique de la lumière, à l'aide d'un appareil du type AMTEC/BI 2030 AT)

### EXEMPLE D

- Cire de Carnauba 20% en poids
- Monostéarate de glycérol oxyéthyléné (300E) vendu sous la dénomination "Tagat S" par la Société Goldschmidt 5,0% en poids
- Alcool stéarylique polyoxyéthyléné vendu sous le nom BRIJ 700 par la société ICI 2% en poids
- Conservateur 0,2% en poids
- Produit résultant de l'oxydation du 5,6-dihydroxyindole 1% en poids
- Tampon KH2PO,/K2HP04 0,5M pH8 10% en poids
- Eau 61,8% en poids

### Etape 1 : Préparation de la microdispersion de cire

Dans un flacon de verre blanc de 250 ml à large ouverture, on introduit les ingrédients suivants
- Cire de Carnauba 48,279 g
- TAGAT S (tensioactif émulsionnant) 12,054 g

On porte à 90°C la cire et l'agent émulsionnant en homogénéisant sous agitation modérée. En continuant d'agiter, on incorpore 149,667 g d'une solution contenant l'eau et le conservateur portée également à 90°C.
La microémulsion obtenue est ramenée à température ambiante et forme une microdispersion de grains à base de cire.
Diamètre moyen des particules de cire : 201nm
Polydispersité en taille des particules : 0,1 (mesurée par diffusion quasi-élastique de la lumière, à l'aide d'un appareil du type AMTEC/BI 2030 AT)

### Etape 2: Stabilisation de la microdispersion de cire

Dans un flacon de verre blanc de 500 ml à large ouverture, on introduit 8,736 g d'agent stabilisant (BRIJ 700) et on complète à 390 g par la microdispersion de cire obtenue dans l'étape 1, refroidie. On solubilise l'agent stabilisant dans la dite dispersion sous agitation magnétique.

La microdispersion de cire obtenue a pour granulométrie
Diamètre moyen des particules de cire 204 nm
Polydispersité en taille des particules 0,1 (mesurée par diffusion quasi-élastique de la lumière, à l'aide d'un appareil du type AMTEC/BI 2030 AT)

### Etape 3 : Préparation de la microdispersion de pigment composite mélanique

### - Solubilisation du précurseur mélanique dans la microdispersion de cire

Dans un flacon de verre blanc de 500 ml à large ouverture, on introduit 4,273 g de 5,6-dihydroxyindole puis on complète à 385 g avec la microdispersion obtenue dans l'étape 2.
On introduit de l'azote dans ledit flacon au dessus du mélange obtenu, ainsi qu'un barreau magnétique et on referme aussitôt le flacon. On solubilise le 5,6 dihydroxyindole sous agitation magnétique pendant 2 heures.

### - Polymérisation in situ du précurseur mélanique dans la microdispersion de cire

On introduit dans un autre flacon de verre blanc de 500 ml à large ouverture, 42 g de solution tampon KH2PO,/K2HP04 0,5M et de pH 8 puis on complète à 420g avec la microdispersion de cire contenant le 5,6-dihydroxyindole. On introduit un barreau magnétique puis on ferme ledit flacon avec une capsule perforée.
On laisse agir le mélange sous agitation magnétique pendant 72 heures jusqu'à la fin de la polymérisation du 5,6-dihydroxyindole.

On obtient une dispersion de particules de pigment composite d'aspect liquide, noir opaque et présentant la granulométrie suivante
Diamètre moyen des particules de cire 206 nm
Polydispersité en taille des particules 0,08 (mesurée par diffusion quasi-élastique de la lumière, à l'aide d'un appareil du type AMTEC/BI 2030 AT)

### EXEMPLE E

- Cire de Carnauba 10% en poids
- Alcool de lanoline et mélange d'alcools gras polyoxyéthylénés (25 OE) vendu sous le nom SOLULAN 25 par la société AMERCHOL 2,5% en poids
- Alcool stéarylique polyoxyéthyléné vendu sous le nom BRIJ 700 par la société ICI 2% en poids
- Produit résultant de l'oxydation du 6- monohydroxyindole 1 % en poids
- Tampon KH2PO4/K2HP04 0,5M pH7 10% en poids
- Eau 74,5% en poids

On procède dans les mêmes conditions des étapes 1 à 3 du procédé de préparation de l'exemple A. On obtient une dispersion de particules de pigment mélanique composite d'aspect liquide, pourpre rouille, opaque et présentant la granulométrie suivante
Diamètre moyen des particules de cire : 115 nm
Polydispersité en taille des particules 0,11 (mesurée par diffusion quasi-élastique de la lumière, à l'aide d'un appareil du type AMTEC/BI 2030 AT)

### EXEMPLE F

- Cire de Carnauba 10% en poids
- Alcool de lanoline et mélange d'alcools gras polyoxyéthylénés (25 OE) vendu sous le nom SOLULAN 25 par la société AMERCHOL 2,5% en poids
- Alcool stéarylique polyoxyéthyléné vendu sous le nom BRIJ 700 par la société ICI 2% en poids
- Produit résultant de l'oxydation du N-méthyl 5,6-dihydroxyindole 1% en poids
- Tampon KH2PO4/K2HP04 0,5M pH7 10% en poids
- Eau 74,5% en poids

On procède dans les mêmes conditions des étapes 1 à 3 du procédé de préparation de l'exemple A. On obtient une dispersion de particules de pigment mélanique composite d'aspect liquide, vert foncé, opaque et présentant la granulométrie suivante :
Diamètre moyen des particules de cire 105 nm
Polydispersité en taille des particules 0,08 (mesurée par diffusion quasi-élastique de la lumière, à l'aide d'un appareil du type AMTEC/BI 2030 AT)

### EXEMPLE G

- Cire de Carnauba 10% en poids
- Alcool de lanoline et mélange d'alcools gras polyoxyéthylénés (25 OE) vendu sous le nom SOLULAN 25 par la société AMERCHOL (tensioactif émulsionnant) 2,5% en poids
- Alcool stéarylique polyoxyéthyléné vendu sous le nom BRIJ 700 par la société ICI (agent stabilisant) 2% en poids
- Produit résultant de l'oxydation du bromhydrate de 5,6-dihydroxyindoline 1% en poids
- Tampon KH2PO4/K2HP04 0,5M pH7 10% en poids
- Eau 74,5% en poids

On procède dans les mêmes conditions des étapes 1 à 3 du procédé de préparation de l'exemple A. On obtient une dispersion de particules de pigment mélanique composite d'aspect liquide, noir avec reflets rougeâtres , opaque et présentant la granulométrie suivante
Diamètre moyen des particules de cire 104 nm
Polydispersité en taille des particules 0,06 (mesurée par diffusion quasi-élastique de la lumière, à l'aide d'un appareil du type AMTEC/BI 2030 AT)

### EXEMPLE H

- Cire de Carnauba 5% en poids
- Alcool cétylique polyoxyéthyléné (20 OE) vendu sous la dénomination BRIJ 58 par la Société ICI (tensioactif émulsionnant) 2,34% en poids
- Alcool stéarylique polyoxyéthyléné vendu sous le nom BRIJ 700 par la société ICI (agent stabilisant) 1 % en poids
- Produit résultant de l'oxydation du 5,6-dihydroxyindole 0,5% en poids
- Eau 91,16% en poids

### Etape 1 : Préparation de la microdispersion de cire

Dans un flacon de verre blanc de 250 ml à large ouverture, on introduit les ingrédients suivants :
- Cire de Carnauba 7,5 g
- BRIJ 58 (tensioactif émulsionnant) 3,51 g
- BRIJ 700 (agent stabilisant) 1,5 g

On porte à 90°C la cire, l'agent émulsionnantet l'agent stabilisant en homogénéisant sous agitation modérée. En continuant d'agiter, on incorpore 134,39 g d'eau portée également à 90°C.
La microémulsion obtenue est ramenée à température ambiante et forme une microdispersion de grains à base de cire.
La microdispersion de cire obtenue a pour granulométrie
Diamètre moyen des particules de cire 50,7nm
Polydispersité en taille des particules 0,17 (mesurée par diffusion quasi-élastique de la lumière, à l'aide d'un appareil du type AMTEC/BI 2030 AT)

### Etape 2 : Préparation de la microdispersion de pigment composite mélanique

### - Solubilisation du précurseur mélanique dans la microdispersion de cire

Les conditions opératoires sont identiques à celles de la solubilisation du précurseur de pigment mélanique dans la microdispersion de cire mises en oeuvre dans le procédé de l'exemple A ou D.

### - Polymérisation in situ du précurseur mélanique dans la microdispersion de cire

La polymérisation par oxydation du 5,6-dihydroxyindole s'effectue avec le système oxydant constitué de :
- peroxyde d'hydrogène (H202) à raison de 5 ml de H202 à 20 volumes par gramme de 5,6-dihydroxyindole et
- iodure de potassium (KI) à raison de 0,4g par gramme de 5,6-dihydroxyindole.
On laisse agir le mélange sous agitation magnétique pendant 22 heures jusqu'à la fin de la polymérisation du 5,6-dihydroxyindole.

On obtient une dispersion de particules de pigment composite d'aspect liquide, noir opaque et présentant la granulométrie suivante
Diamètre moyen des particules de cire 54 nm
Polydispersité en taille des particules 0,19 (mesurée par diffusion quasi-élastique de la lumière, à l'aide d'un appareil du type AMTEC/BI 2030 AT)

### - Test d'inhibition de la production d'éthylène par la formulation de l'exemple H

Dans une boîte de Pétri de diamètre 32 mm, sont introduits dans l'ordre suivant
- 1,4 ml de tampon phosphate 50 mM (pH = 7,4),
- 100 µl de solution 200 mM de méthionine,
- 100 µl de solution 4 mM de chlorure ferrique,
- 100 µl du produit à tester,
- 100 µl de solution 4 mM d'EDTA (acide éthylènediamine tétracétique),
- 100 µl de solution 400 mM de N.A.D.H. (nicotinamide adénine dinucléotide, forme réduite),
- 100 µl de solution 2 mM de riboflavine.

L'échantillon a un volume total de 2 ml.

Cette boîte est ensuite placée sur une coupelle en aluminium et recouverte d'une cellule en quartz afin d'être exposée sous les U.V.A. (365 nm) à la dose d'1 J/cm2. L'ensemble composé de N.A.D.H., de riboflavine, de chlorure ferrique et d'E.D.T.A., soumis à l'exposition des U.V.A., va générer des espèces réduites de l'oxygène : 0₂^{o-}, H₂O₂, et principalement le radical hydroxyle °OH. Ce dernier va réagir avec la méthionine en libérant de l'éthylène dont la quantité est mesurée par chromatographie en phase gazeuse.

Plus la quantité de radicaux libres formés est grande, plus est importante la quantité d'éthylène libéré. Les résultats sont exprimés en pourcentage de pouvoir inhibiteur correspondant au pourcentage de diminution de la production d'éthylène par rapport au témoin (contenant 100 µl de tampon phosphate remplaçant le produit à tester).

La composition de l'exemple H permet de réduire de 90% la production d'éthylène et présente donc un bon pouvoir inhibiteur sur la formation des radicaux °OH.

### Exemples de formulations cosmétiques

### EXEMPLE 1 : Mascara

On prépare la composition suivante
- Microdispersion de pigment composite (exemple D) 89,5 g
- Hydroxyéthylcellulose vendue sous la dénomination de "Cellosize QP 440OM" par la Société AMERCHOL 1 g
- Gomme arabique 1,5 g
- Panthénol 1,0 g
- NaOH qs pH 7
- Eau 7,0 g

### Mode opératoire:

On opère par dilution de la microdispersion selon l'exemple D en 2 étapes. A température ambiante, le ou les polymères présents dans la formulation sont incorporés dans la microdispersion selon l'exemple D sous agitation en ajoutant éventuellement de l'eau, si nécessaire, pour obtenir une préparation homogène. Ensuite, on disperse les pigments. La formulation peut être broyée.
On applique cette composition sur les cils. A l'application, on constate que les cils sont brillants, possèdent un bon recourbement et une bonne souplesse.

### EXEMPLE 2: Mascara

- Microdispersion de pigment composite selon l'exemple D 86 g
- Glycérine 3 g
- Polyvinylpyrrolidone vendue sous la dénomination de "Luviskol K90" par la Société BASF 4 g
- Polyméthacrylate de sodium 1 g
- NaOH qs pH 7
- Eau 7,0 g

On prépare cette formulation dans les mêmes conditions que l'exemple 1. On applique cette composition sur les cils. A l'application, on constate que les cils sont brillants, possèdent un bon recourbement et une bonne souplesse.

### EXEMPLE 3: Crème solaire

### Phase grasse 1

- PEG-100 stéarate/glycéryl stéarate vendu sous la dénomination ARLACEL 165 par ICI 3 g
- Acide stéarique vendu sous la dénomination STEARINE TP par STEARINERIE DUBOIS 1 g
- Octyldodécanol vendu sous la dénomination EUTANOL G par HENKEL 5 g
- Cyclométhicone vendu sous la dénomination DC 244 FLUID par DOVV CORNING 7 9

### Phase aqueuse 2 (gel) :

- Poly(acide acrylique) vendu sous la dénomination CARBOPOL 940 par GOODRICH 0,25 g
- Triéthanolamine 1,3 g
- Propylèneglycol 2,5 g
- Conservateurs qs
- Eau déminéralisée qsp 100 g

### Phase 3 :

- Microdispersion de pigment composite selon l'un des exemples A, B, C, E, F et G 1 g

### Mode opératoire

On prépare la phase grasse 1 par simples pesée et mélange des ingrédients cités ci-dessus.
On prépare la phase aqueuse 2 (gel) en dispersant le CARBOPOL 940 dans le mélange eau/propylèneglycol préalablement chauffé à 80)C, sous agitation avec un mélangeur du type MORITZ jusqu'à homogénéisation complète puis on neutralise jusqu'à pH 6-7 avec la triéthanolamine. On obtient ainsi un gel. On fait chauffer la phase grasse à 80°C et on ajoute le gel aqueux obtenu puis on mélange sous agitation du type MORITZ. On laisse refroidir jusqu'à 45°C environ et on ajoute et disperse les conservateurs sous agitation du type MORITZ. On introduit enfin la microdispersion de cire (phase 3) à température ambiante que l'on disperse dans le mélange, sous agitation jusqu'à homogénéisation complète.

## Revendications

1. Pigment mélanique composite sous forme de particules, **caractérisé par le fait que** lesdites particules sont constituées d'un noyau sphérique de diamètre inférieur à 1 µm comprenant au moins une cire et au moins un tensioactif émulsionnant et d'une couche externe enveloppant ledit noyau comprenant au moins un composé résultant de la polymérisation oxydative d'un précurseur de pigment de type mélanique.

2. Pigment selon la revendication 1, où la cire présente dans le noyau des particules de pigment composite représente de 20 à 97% en poids et plus préférentiellement de 40 à 85% du poids total du noyau.

3. Pigment selon l'une des revendications 1 et 2, où le noyau des particules comprend une cire ou un mélange de cires dont le point de fusion varie de 50 à 100°C.

4. Pigment selon l'une quelconque des revendications 1 à 3, où le noyau des particules comprend au moins une cire ou un mélange de cires choisis dans le groupe constitué par la cire de Carnauba, la cire de Candellila, la cire d'Alfa et leurs mélanges.

5. Pigment selon la revendication 4, où le noyau des particules comprend au moins 20% en poids et en particulier au moins 50% en poids de ladite cire ou dudit mélange de cires par rapport à la quantité totale de cire présente dans ledit noyau.

6. Pigment selon la revendication 4 ou 5, où le noyau des particules comprend en plus une cire ou un mélange de cires choisis dans le groupe constitué par la cire de paraffine; l'ozokérite ; les cires végétales; les cires animales; les cires marines; les céramides naturels ou synthétiques; les cires de polyéthylène.

7. Pigment selon l'une quelconque des revendications 1 à 6, où le noyau des particules contient en plus un ou plusieurs additifs gras huileux ou pâteux pouvant représenter jusqu'à 30% en poids du poids total du noyau.

8. Pigment selon l'une quelconque des revendications 1 à 7, où le noyau des particules contient en plus un ou plusieurs actifs liposolubles pouvant représenter jusqu'à 30% en poids et plus préférentiellement jusqu'à 10% en poids du poids total du noyau.

9. Pigment selon l'une quelconque des revendications 1 à 8, où le noyau comprend de 3 à 50% en poids et plus particulièrement de 10 à 30% en poids de tensioactif émulsionnant par rapport au poids total du noyau.

10. Pigment selon l'une quelconque des revendications 1 à 9, où le rapport pondéral cire(s)/tensioactif(s) varie de préférence de 1 à 30 et plus particulièrement de 2 à 10.

11. Pigment selon l'une quelconque des revendications 1 à 10, où le noyau des particules de pigment composite comporte en plus au moins un composé amphiphile non-ionique qui présente une chaîne hydrocarbonée linéaire ou ramifiée, saturée ou insaturée, aliphatique ou aromatique et au moins 50 moles de motifs polaires ou hydrophiles constituant la tête polaire.

12. Pigment selon la revendication 11 dans lequel le composé amphiphile non ionique est choisi dans le groupe constitué par : les acides gras ou les amides polyalcoxylés et/ou polyglycérolés, les esters d'acide gras et de polyols polyalcoxylés et/ou polyglycérolés, les alcools gras ou les alkylphénols polyalcoxylés et/ou polyglycérolés, les alcanediols ou alcènediols-1,2 ou 1,3 polyalcoxylés et/ou polyglycérolés, les thioéthers de polyacrylamide et de dodécyle, les dérivés alcoxylés des acides gras ou des alcool gras de la lanoline comportant au moins 50 moles de motifs polaires ou hydrophiles constituant la tête polaire.

13. Pigment selon l'une quelconque des revendications 11 à 12, où les composés amphiphiles non-Ioniques répondent à la formule suivante
RO-(CH₂CH₂O)ₙ-H
dans laquelle R désigne un radical alkyle, alcényle ou aryle comportant au moins 8 atomes de carbone et n est un nombre allant de 70 à 200 et plus particulièrement de 100 à 200.

14. Pigment selon l'une quelconque des revendications 11 à 13, où les composés amphiphiles non-ioniques sont présents dans des concentrations allant de 1 à 40% en poids et de préférence de 5 à 20% en poids par rapport au poids total du noyau cireux et où le rapport pondéral tensioactif(s) émulsionnant(s)/composé(s) amphiphile(s) non-ionique(s) varie de 1 à 50 et plus particulièrement de 1 à 20.

15. Pigment selon l'une quelconque des revendications 1 à 14, où le précurseur de pigment mélanique est choisi dans le groupe constitué par
(i) les composés indoliniques de formule suivante et leurs sels d'addition avec un acide 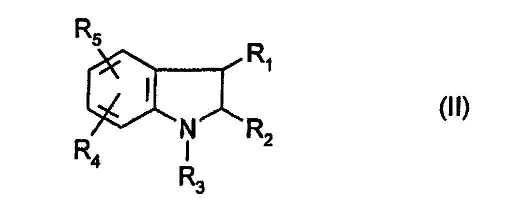 dans laquelle:
R1 et R3 représentent, indépendamment l'un de l'autre, un atome d'hydrogène ou un groupement alkyle en C1-C4;
R2 désigne un atome d'hydrogène, un groupement alkyle en C1-C4, un groupement carboxyle, ou alcoxy (C1-C4) carbonyle;
R4 désigne un atome d'hydrogène, un groupement alkyle en C1-C4, un groupement hydroxyle, un groupement alcoxy (C1-C4) amino, un groupement alkyl (C1-C10) amino ou un atome d'halogène ;
R5 désigne un atome d'hydrogène, un groupement alkyle en C1-C4, un groupement hydroxyle, un groupement alcoxy en C1-C4 ou amino ;
au moins l'un des radicaux R4 ou R5 désignant un groupe hydroxyle, alcoxy, ou amino; sous réserve que lorsque R5 désigne amino alors R4 est différent d'un radical alkylamino ;
R4 et R5 peuvent former un cycle alkylène dioxy et sont en position 5 et 6.
(ii) les composés indoliques de formule suivante et leurs sels d'addition avec un acide : 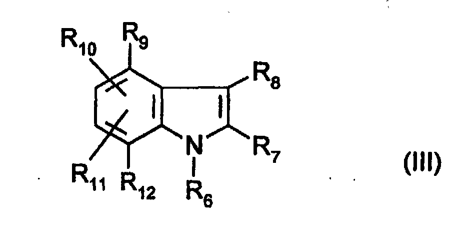 dans laquelle
R6 et R8 représentent, indépendamment l'un de l'autre, un atome d'hydrogène ou un groupement alkyle en C1-C4;
R7 désigne un atome d'hydrogène, un groupement alkyle en C1-C4, un groupement carboxyle, ou alcoxy (C1-C4) carbonyle ;
R9 et R12 désignent, indépendamment l'un de l'autre, un atome d'hydrogène, un groupement alkyle en C1-C4, un groupement hydroxyle, un groupement alcoxy en C1-C4, amino, un groupement acyl (C2-C4)oxy, acyl (C2-C4)amino ;
R10 désigne un atome d'hydrogène, un groupement alkyle en C1-C4, un groupement hydroxyle, un groupement alcoxy en C1-C4, amino, un groupement acyl (C2-C4)oxy, acyl (C2-C4)amino, un atome d'halogène ou un groupe triméthylsilyloxy;
R11 désigne un atome d'hydrogène, un groupement hydroxyle, un groupement alcoxy en C1-C4, amino, un groupement acyl(C2-C4)oxy, acyl(C2-C4)amino, hydroxyalkyl(C2-C4)amino ou un groupe triméthylsilyloxy ;
R10 et R11 peuvent former, conjointement avec les atomes de carbone auxquels ils sont attachés, un cycle méthylènedioxy éventuellement substitué par un alkyle en C1-C4, un alcoxy en C1-C4 ou un cycle carbonyldioxy;
au moins l'un des groupements R9 à R12 représente un groupe OZ ou NHR et un seul desdits groupements désigne NHR;
au plus deux des groupements R9 à R12 représente un groupe OZ, lorsque Z désigne hydrogène et sont en position 5 et 6;
au moins l'un des groupements R9 à R12 désigne hydrogène, dans le cas où un seul de ces groupements désignent hydrogène, un seul desdits groupement désigne un groupe NHR ou OZ et les autres désignent un groupe alkyle en C1-C4;
R désigne un atome d'hydrogène, un groupe acyle en C2-C4, un groupe hydroxyalkyle en C2-C4 ;
Z désigne un atome d'hydrogène, un groupe acyle en C2-C14, un groupe alkyle en C1-C4, triméthylsilyle.
(iii) leurs mélanges.

16. Pigment selon l'une quelconque des revendications 1 à 15, où le précurseur de pigment mélanique est choisi dans le groupe constitué par la 5,6dihydroxyindoline, le 6-hydroxyindole, le 5,6-dihydroxyindole et le N-méthyl 5,6dihydroxyindole ainsi que leurs sels d'addition avec un acide.

17. Pigment selon l'une quelconque des revendications 1 à 16, où le ou les précurseur(s) de pigment mélanique est ou sont utilisés dans des quantités telles que le rapport pondéral cire(s)/colorant formé in situ après oxydation soit supérieur ou égal à 3.

18. Pigment selon l'une quelconque des revendications 1 à 17, où la taille des particules sphériques de pigment mélanique composite est inférieure à 1 µm et plus préférentiellement inférieure ou égale à 500 nm et plus particulièrement varie de 20 à 500 nm.

19. Procédé de préparation de particules de pigment mélanique composite telles que définies selon l'une quelconque des revendications 1 à 18, **caractérisé en ce qu'**il consiste à disperser et solubiliser, à température ambiante, sous agitation douce et sous atmosphère inerte, un précurseur de type mélanique tel que défini selon l'une quelconque des revendications 1, 14 à 16 dans une dispersion aqueuse de particules sphériques de cire de dimension inférieure à 1 µm contenant en plus au moins un tensioactif émulsionnant telles que celles définies ci-dessus selon l'une quelconque des revendications 1 à 14, puis à oxyder ledit précurseur de manière à le polymériser et à former une couche externe enveloppant chaque particule de cire puis à éventuellement sécher la dispersion aqueuse de particules de pigment mélanique composite ainsi obtenue de manière à obtenir une poudre.

20. Procédé selon la revendication 19, où l'étape d'oxydation s'effectue en dessous de la température de fusion de la cire ou du mélange de cires constituant les particules de cire et de préférence à température ambiante.

21. Procédé selon la revendication 19 ou 20, où l'étape de polymérisation s'effectue en milieux aqueux par auto-oxydation à l'air provoquée par une agitation vigoureuse de la microdispersion de cire ajustée à un pH neutre ou alcalin.

22. Procédé selon la revendication 19 ou 20, où l'étape de polymérisation s'effectue en présence d'un agent oxydant choisi dans le groupe constitué par le peroxyde d'hydrogène, les peracides et les persels et éventuellement en présence d'un modificateur de pH.

23. Procédé selon la revendication 19 ou 20, où l'étape de polymérisation s'effectue en présence d'un agent oxydant choisi dans le groupe constitué par les chlorites alcalins, l'oxyde d'argent, le chlorure ferrique, l'oxyde de plomb, le nitrite de sodium ; les sels de terres, les ortho et parabenzoquinones, les ortho et parabenzoquinones monoimines et diimines, les 1,2- et 1,4-naphtoquinones, les 1,2 et les 1,4-naphtoquinones mono- ou diimines et éventuellement en présence d'un modificateur de pH.

24. Procédé selon la revendication 19 ou 20, où l'étape de polymérisation s'effectue par utilisation d'un ion iodure en présence de peroxyde d'hydrogène et éventuellement en présence d'un modificateur de pH.

25. Procédé selon la revendication 19 ou 20, où l'étape de polymérisation s'effectue par oxydation par voie enzymatique.

26. Procédé selon l'une quelconque des revendications 19 à 25, où l'on incorpore à chaud ou à froid, un composé amphiphile non-ionique à longue tête polaire tel que défini selon l'une quelconque des revendications 11 à 14 dans la microdispersion aqueuse de cire avant de disperser dans celle-ci le précurseur de pigment mélanique.

27. Utilisation d'un pigment mélanique composite sous forme de particules sphériques telles que définies dans l'une quelconque des revendications 1 à 18, à l'état de poudre ou en dispersion aqueuse, dans et pour la préparation d'une composition cosmétique ou dermatologique.

28. Composition cosmétique ou dermatologique comprenant au moins un pigment mélanique composite sous forme de particules sphériques à l'état de poudre ou en dispersion aqueuse telles que définies dans l'une quelconque des revendications 1 à 18.

29. Composition selon la revendication 28, où le pigment mélanique composite, est utilisé sous forme de poudre ou bien en dispersion aqueuse à une concentration allant de 0,1 à 35% en poids et en particulier de 0,5 à 20 % en poids par rapport au poids total de la composition.

30. Utilisation d'un pigment mélanique composite sous forme de particules sphériques telles que définies dans l'une quelconque des revendications 1 à 18, à l'état de poudre ou en dispersion aqueuse, dans et pour la préparation de produits de maquillage de la peau et/ou des phanères.

31. Utilisation d'un pigment mélanique composite sous forme de particules sphériques telles que définies dans l'une quelconque des revendications 1 à 18, à l'état de poudre ou en dispersion aqueuse, dans et pour la préparation de compositions tinctoriales pour cheveux, notamment pour réaliser une teinture temporaire ou un maquillage des cheveux.

32. Utilisation d'un pigment mélanique composite sous forme de particules sphériques telles que définies dans l'une quelconque des revendications 1 à 18, à l'état de poudre ou en dispersion aqueuse, dans et pour la préparation de compositions protectrices de l'épiderme humain et/ou des phanères contre les effets néfastes des rayons UV en particulier de produits dits solaires.

33. Utilisation d'un pigment mélanique composite sous forme de particules sphériques telles que définies dans l'une quelconque des revendications 1 à 18, à l'état de poudre ou en dispersion aqueuse, dans et pour la préparation de compositions protectrices de la peau et/ou des phanères contre les effets des radicaux libres induits par les polluants atmosphériques et/ou par le rayonnement ultraviolet.

## Patentansprüche

1. Melaninverbundpigment in Form von Partikeln, **dadurch gekennzeichnet, daß** die Partikel aus einem kugelförmigen Kern mit einem Durchmesser unter 1 µm, der mindestens ein Wachs und mindestens einen emulgierenden grenzflächenaktiven Stoff enthält, und einer äußeren Schicht bestehen, die den Kern umhüllt, die mindestens eine Verbindung enthält, die bei der oxidativen Polymerisation eines Vorläufers eines Pigments vom Melanintyp entsteht.

2. Pigment nach Anspruch 1, wobei das Wachs, das in dem Kern der Verbundpigment-Partikel enthalten ist, 20 bis 97 Gew.-% und noch bevorzugter 40 bis 85 % des Gesamtgewichts des Kerns ausmacht.

3. Pigment nach einem der Ansprüche 1 und 2, wobei der Kern der Partikel ein Wachs oder Wachsgemisch enthält, dessen Schmelzpunkt im Bereich von 50 bis 100 °C liegt.

4. Pigment nach einem der Ansprüche 1 bis 3, wobei der Kern der Partikel mindestens ein Wachs oder Wachsgemisch enthält, das unter Carnaubawachs, Candellilawachs, Alfa-Wachs und den Gemischen dieser Wachse ausgewählt ist.

5. Pigment nach Anspruch 4, wobei der Kern der Partikel mindestens 20 Gew.-% und vor allem mindestens 50 Gew.-% dieses Wachses oder Wachsgemischs enthält, bezogen auf die Gesamtmenge an Wachsen, die in dem Kern enthalten sind.

6. Pigment nach Anspruch 4 oder 5, wobei der Kern der Partikel außerdem ein Wachs oder Wachsgemisch enthält, das unter Paraffinwachs, Ozokerit, pflanzlichen Wachsen, tierischen Wachsen, marinen Wachsen, natürlichen oder synthetischen Ceramiden, Polyethylenwachsen ausgewählt ist.

7. Pigment nach einem der Ansprüche 1 bis 6, wobei der Kern der Partikel zusätzlich ein oder mehrere ölige oder pastöse Fettsubstanzen als Zusatzstoffe enthält, die bis zu 30 Gew.-% des Gesamtgewichts des Kerns ausmachen können.

8. Pigment nach einem der Ansprüche 1 bis 7, wobei der Kern der Partikel außerdem ein oder mehrere fettlösliche Wirkstoffe enthält, die bis zu 30 Gew.-% und noch bevorzugter bis zu 10 Gew.-% des Gesamtgewichts des Kerns ausmachen können.

9. Pigment nach einem der Ansprüche 1 bis 8, wobei der Kern 3 bis 50 Gew.-% und vor allem 10 bis 30 Gew.-% emulgierende grenzflächenaktive Stoffe, bezogen auf das Gesamtgewicht des Kerns, enthält.

10. Pigment nach einem der Ansprüche 1 bis 9, wobei das Gewichtsverhältnis des oder der Wachse zu dem oder den grenzflächenaktiven Stoffen vorzugsweise im Bereich von 1 bis 30 und noch bevorzugter im Bereich von 2 bis 10 liegt.

11. Pigment nach einem der Ansprüche 1 bis 10, wobei der Kern der Verbundpigment-Partikel außerdem mindestens eine nichtionische amphiphile Verbindung enthält, die eine geradkettige oder verzweigte, gesättigte oder ungesättigte, aliphatische oder aromatische Kohlenwasserstoffkette und mindestens 50 mol polare oder hydrophile Einheiten, die den polaren Kopf bilden, aufweist.

12. Pigment nach Anspruch 11, wobei die nichtionische amphiphile Verbindung ausgewählt ist unter: den polyalkoxylierten und/oder mehrfach mit Glycerin veretherten Fettsäuren oder Amiden, den polyalkoxylierten und/oder mehrfach mit Glycerin veretherten Estern aus einer Fettsäure und Polyolen, den polyalkoxylierten und/oder mehrfach mit Glycerin veretherten Fettalkoholen oder Alkylphenolen, den polyalkoxylierten und/oder mehrfach mit Glycerin veretherten 1,2- oder 1,3-Alkandiolen oder 1,2- oder 1,3-Alkendiolen, den Thioethern von Polyacrylamid und von Dodecyl, den alkoxylierten Derivaten der Fettsäuren oder Fettalkohole von Lanolin, die mindestens 50 mol polare oder hydrophile Einheiten enthalten, die den polaren Kopf bilden.

13. Pigment nach einem der Ansprüche 11 und 12, wobei die nichtionischen amphiphilen Verbindungen der folgenden Formel
RO-(CH₂CH₂O)ₙ-H
entsprechen, in der R eine Alkyl-, Alkenyl- oder Arylgruppe bedeutet, die mindestens 8 Kohlenstoffatome enthält, und n eine Zahl ist, die im Bereich von 70 bis 200 und vor allem 100 bis 200 liegt.

14. Pigment nach einem der Ansprüche 11 bis 13, wobei die nichtionischen amphiphilen Verbindungen in Konzentrationen im Bereich von 1 bis 40 Gew.-% und vorzugsweise 5 bis 20 Gew.-%, bezogen auf das Gesamtgewicht des wachshaltigen Kerns, enthalten sind und wobei das Gewichtsverhältnis des oder der emulgierenden grenzflächenaktiven Stoffe zu der oder den nichtionischen amphiphilen Verbindungen im Bereich von 1 bis 50 und vor allem 1 bis 20 liegt.

15. Pigment nach einem der Ansprüche 1 bis 14, wobei der Vorläufer des Melaninpigments ausgewählt ist unter
(i) den Indolinverbindungen der folgenden Formel und ihren Additionssalzen mit einer Säure 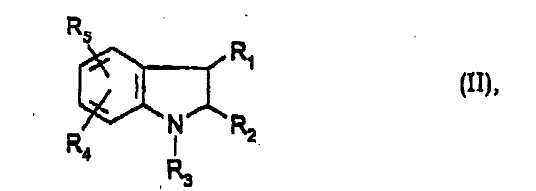 in der
• R₁ und R₃ unabhängig voneinander ein Wasserstoffatom oder eine C₁-C₄-Alkylgruppe darstellen,
• R₂ ein Wasserstoffatom, eine C₁-C₄-Alkylgruppe, eine Carboxygruppe oder (C₁-C₄)-Alkoxycarbonylgruppe bedeutet,
• R₄ ein Wasserstoffatom, eine (C₁-C₄)-Alkylgruppe, eine Hydroxygruppe, eine (C₁-C₄)-Alkoxyaminogruppe, eine (C₁-C₁₀)-Alkylaminogruppe oder ein Halogenatom bedeutet, und R₅ ein Wasserstoffatom, eine C₁-C₄-Alkylgruppe, eine Hydroxygruppe, eine C₁-C₄-Alkoxygruppe oder Amino bedeutet, wobei mindestens eine der Gruppen R₄ und R₅ eine Hydroxy, Alkoxy- oder Aminogruppe bedeutet; mit der Maßgabe, daß R₄ verschieden von Alkylamino ist, wenn R₅ Amino bedeutet,
• R₄ und R₅ einen Alkylendioxy-Ring bilden können,und sich in Position 5 und 6 befinden;
(ii) den Indolverbindungen der folgenden Formel und ihren Additionssalzen mit einer Säure 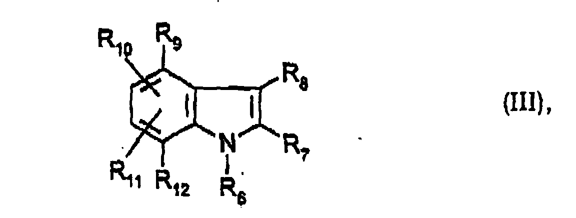 in der
• R₆ und R₈ unabhängig voneinander ein Wasserstoffatom oder eine C₁-C₄-Alkylgruppe darstellen,
• R₇ ein Wasserstoffatom, eine C₁-C₄-Alkylgruppe, eine Carboxygruppe oder (C₁-C₄)-Alkoxycarbonylgruppe bedeutet,
• R₉ und R₁₂ unabhängig voneinander ein Wasserstoffatom, eine C₁-C₄-Alkylgruppen, eine Hydroxygruppe, eine C₁-C₄-Alkoxygruppe, Amino, eine (C₂-C₄)-Acyloxygruppe, eine (C₂-C₄)-Acylaminogruppe bedeuten,
• R₁₀ ein Wasserstoffatom, eine (C₁-C₄)-Alkylgruppe, eine Hydroxygruppe, eine (C₁-C₄)-Alkoxygruppe, Amino, eine (C₂-C₄)-Acyloxygruppe, eine (C₂-C₄)-Acylaminogruppe, ein Halogenatom oder eine Trimethylsilyloxygruppe bedeutet,
• R₁₁ ein Wasserstoffatom, eine Hydroxygruppe, eine C₁-C₄-Alkoxygruppe, eine Aminogruppe, eine (C₂-C₄)-Acyloxygruppe, eine (C₂-C₄)-Acylaminogruppe, eine (C₂-C₄)-Hydroxyalkylaminogruppe oder eine Trimethylsilyloxygruppe bedeutet;
• R₁₀ und R₁₁ zusammen mit den Kohlenstoffatomen, mit denen sie verbunden sind, einen Methylendioxyring bilden können, der gegebenenfalls mit C₁-C₄-Alkyl, C₁-C₄-Alkoxy oder einem Carbonyldioxy-Ring substituiert ist;
• mindestens eine der Gruppen R₉ bis R₁₂ eine Gruppe OZ oder NHR darstellt und nur eine dieser Gruppen NHR bedeutet;
• höchstens zwei der Gruppen R₉ bis R₁₂ eine Gruppe OZ bedeuten und sich in Position 5 und 6 befinden, wenn Z Wasserstoff darstellt;
• mindestens eine der Gruppen R₉ bis R₁₂ Wasserstoff bedeutet in dem Fall, daß eine einzige dieser Gruppen Wasserstoff bedeutet, eine einzige dieser Gruppen eine Gruppe NHR oder OZ bedeutet und die anderen eine C₁-C₄-Alkylgruppe bedeuten;
• R ein Wasserstoffatom, eine C₂-C₄-Acylgruppe, eine C₂-C₄-Hydroxyalkylgruppe bedeutet;
• Z ein Wasserstoffatom, eine C₂-C₁₄-Acylgruppe, eine C₁-C₄-Alkylgruppe, Trimethylsilyl bedeutet,
(iii) ihren Gemischen.

16. Pigment nach einem der Ansprüche 1 bis 15, wobei der Vorläufer des Melaninpigments unter 5,6-Dihydroxyindolin, 6-Hydroxyindol, 5,6-Dihydoxyindol und N-Methyl-5,6-dihydroxyindol sowie ihren Additionssalzen mit einer Säure ausgewählt ist.

17. Pigment nach einem der Ansprüche 1 bis 16, wobei der oder die Vorläufer des Melaninpigments in solchen Mengen verwendet wird/werden, daß das Gewichtsverhältnis des oder der Wachse zu dem *in situ* nach der Oxidation gebildeten Farbstoff größer als oder gleich 3 ist.

18. Pigment nach einem der Ansprüche 1 bis 17, wobei die Größe der kugelförmigen Melaninverbundpigment-Partikel unter 1 µm und noch bevorzugter bei 500 nm oder darunter und vor allem im Bereich von 20 bis 500 nm liegt,

19. Verfahren zur Herstellung der Melaninverbundpigment-Partikel, die wie in einem der Ansprüche 1 bis 18 definiert sind, **dadurch gekennzeichnet, daß** es darin besteht, bei Umgebungstemperatur, unter leichtem Rühren und unter einer inerten Atmosphäre einen Vorläufer vom Melanintyp, der wie in einem der Ansprüche 1, 14 bis 16 definiert ist, in einer wäßrigen Dispersion kugelförmiger Wachspartikel mit einer Größe unter 1 µm, die zusätzlich mindestens einen emulgierenden grenzflächenaktiven Stoff enthalten, die wie in einem der Ansprüche 1 bis 14 definiert sind, zu dispergieren und zu solubilisieren, anschließend diesen Vorläufer zu oxidieren, um ihn zu polymerisieren und eine äußere Schicht zu bilden, die jedes Wachspartikel umhüllt, und dann gegebenenfalls die so erhaltene wäßrige Dispersion der Melaninverbundpigment-Partikel zu trocknen, um ein Pulver zu erhalten.

20. Verfahren nach Anspruch 19 wobei der Oxidationsschritt unterhalb der Schmelztemperatur des Wachses oder des Gemischs von Wachsen, aus dem die Wachspartikel bestehen, und vorzugsweise bei Umgebungstemperatur durchgeführt wird.

21. Verfahren nach Anspruch 19 oder 20, wobei der Polymerisationsschritt in wäßrigem Medium durch Autoxidation an der Luft durchgeführt wird, die durch kräftiges Rühren der Mikrodispersion des Wachses, die auf einen neutralen oder alkalischen pH-Wert eingestellt ist, hervorgerufen wird.

22. Verfahren nach Anspruch 19 oder 20, wobei der Polymerisationsschritt in Gegenwart eines Oxidationsmittels, das unter Wasserstoffperoxid, den Persäuren und den Persalzen ausgewählt wird, und gegebenenfalls in Gegenwart eines den pH-Wert modifizierenden Mittels durchgeführt wird.

23. Verfahren nach Anspruch 19 oder 20, wobei der Polymerisationsschritt in Gegenwart eines Oxidationsmittels, das unter den Alkalichloriten, Silberoxid, Eisen(III)-chlorid, Bleioxid, Natriumnitrit; den Salzen von Seltenerdmetall, o- und p-Benzochinon, o- und p-Benzochinonmonoimin und o- und p-Benzochinondiimin, 1,2- und 1,4-Naphthochinon, 1,2- und 1,4-Naphthochinonmonoimin und 1,2-- und 1,4-Naphthochinondiimin ausgewählt wird, und gegebenenfalls in Gegenwart eines den pH-Wert modifizierenden Mittels durchgeführt wird.

24. Verfahren nach Anspruch 19 oder 20, wobei der Polymerisationsschritt unter Verwendung eines Iodid-Ions in Gegenwart von Wasserstoffperoxid und gegebenenfalls in Gegenwart eines den pH-Wert modifizierenden Mittels durchgeführt wird.

25. Verfahren nach Anspruch 19 oder 20, wobei der Polymerisationsschritt durch Oxidation auf enzymatischem Weg durchgeführt wird.

26. Verfahren nach einem der Ansprüche 19 bis 25, wobei in der Hitze oder in der Kälte eine nichtionische amphiphile Verbindung mit langem polarem Kopf, die wie in einem der Ansprüche 11 bis 14 definiert ist, in die wäßrige Mikrodispersion des Wachses eingebracht wird, bevor der Vorläufer des Melaninpigments in der Mikrodispersion dispergiert wird.

27. Verwendung eines Melaninverbundpigments in Form von kugelförmigen Partikeln, die wie in einem der Ansprüche 1 bis 18 definiert sind, in Form eines Pulvers oder in wäßriger Dispersion, in und für die Herstellung einer kosmetischen oder dermatologischen Zusammensetzung.

28. Kosmetische oder dermatologische Zusammensetzung, die mindestens ein Melaninverbundpigment in Form von kugelförmigen Partikeln, die wie in einem der Ansprüche 1 bis 18 definiert sind, in Form eines Pulvers oder in wäßriger Dispersion enthält.

29. Zusammensetzung nach Anspruch 28, wobei das Melaninverbundpigment in Form eines Pulvers oder in wäßriger Dispersion in einer Konzentration verwendet wird, die im Bereich von 0,1 bis 35 Gew.-% und insbesondere 0,5 bis 20 Gew.-%, bezogen auf das Gesamtgewicht der Zusammensetzung, liegt.

30. Verwendung eines Melaninverbundpigments in Form von kugelförmigen Partikeln, die wie in einem der Ansprüche 1 bis 18 definiert sind, in Form eines Pulvers oder in wäßriger Dispersion, in und für die Herstellung von Produkten zum Schminken der Haut und/oder der Hautanhangsgebilde.

31. Verwendung eines Melaninverbundpigments in Form von kugelförmigen Partikeln, die wie in einem der Ansprüche 1 bis 18 definiert sind, in Form eines Pulvers oder in wäßriger Dispersion, in und für die Herstellung von Färbemittelzusammensetzungen für die Haare, insbesondere für die Erzeugung einer temporären Färbung oder zum Schminken der Haare.

32. Verwendung eines Melaninverbundpigments in Form von kugelförmigen Partikeln, die wie in einem der Ansprüche 1 bis 18 definiert sind, in Form eines Pulvers oder in wäßriger Dispersion in und für die Herstellung von Zusammensetzungen für den Schutz der menschlichen Epidermis und/oder der Hautanhangsgebilde vor den schädlichen Wirkungen von UV-Strahlung, insbesondere von Sonnenschutzprodukten.

33. Verwendung eines Melaninverbundpigments in Form von kugelförmigen Partikeln, die wie in einem der Ansprüche 1 bis 18 definiert sind, in Form eines Pulvers oder in wäßriger Dispersion, in und für die Herstellung von Zusammensetzungen für den Schutz der Haut und/oder der Hautanhangsgebilde vor den Auswirkungen freier Radikale, die durch Verunreinigungen in der Atmosphäre und/oder durch UV-Strahlung hervorgerufen werden.

## Claims

1. Composite melanin pigment in the form of particles, **characterized in that** the said particles consist of a spherical core less than 1 µm in diameter, comprising at least one wax and at least one emulsifying surfactant, and of an outer layer which envelops the said core, comprising at least one compound resulting from the oxidative polymerization of a melanin-type pigment precursor.

2. Pigment according to Claim 1, in which the wax present in the core of the composite pigment particles represents from 20 to 97% by weight, and more preferably from 40 to 85%, of the total weight of. the core.

3. Pigment according to either of Claims 1 and 2, in which the core of the particles comprises a wax or a wax mixture whose melting point ranges from 50 to 100°C.

4. Pigment according to any one of Claims 1 to 3, in which the core of the particles comprises at least one wax or a wax mixture chosen from the group consisting of carnauba wax, candelilla wax, alfalfa wax and mixtures thereof.

5. Pigment according to Claim 4, in which the core of the particles comprises at least 20% by weight, and in particular at least 50% by weight, of the said wax or of the said wax mixture relative to the total amount of wax present in the said core.

6. Pigment according to Claim 4 or 5, in which the core of the particles also comprises a wax or a wax mixture chosen from the group consisting of paraffin wax; ozokerite; plant waxes; animal waxes; marine waxes; natural or synthetic ceramides; polyethylene waxes.

7. Pigment according to any one of Claims 1 to 6, in which the core of the particles also contains one or more oily or pasty fatty additives which can represent up to 30% by weight of the total weight of the core.

8. Pigment according to any one of Claims 1 to 7, in which the core of the particles also contains one or more liposoluble active agents which can represent up to 30% by weight, and more preferably up to 10% by weight, of the total weight of the core.

9. Pigment according to any one of Claims 1 to 8, in which the core comprises from 3 to 50% by weight, and more particularly from 10 to 30% by weight, of emulsifying surfactant relative to the total weight of the core.

10. Pigment according to any one of Claims 1 to 9, in which the wax(es)/surfactant(s) weight ratio preferably ranges from 1 to 30 and more particularly from 2 to 10.

11. Pigment according to any one of Claims 1 to 10, in which the core of the composite pigment particles also contains at least one nonionic amphiphilic compound which has a linear or branched, saturated or unsaturated, aliphatic or aromatic hydrocarbon chain and at least 50 mol of polar or hydrophilic units which constitute the polar head.

12. Pigment according to Claim 11, in which the nonionic amphiphilic compound is chosen from the group consisting of: polyalkoxylated and/or polyglycerolated fatty acids or amides, polyalkoxylated and/or polyglycerolated fatty acid esters of polyols, polyalkoxylated and/or polyglycerolated alkylphenols or fatty alcohols, polyalkoxylated and/or polyglycerolated 1,2- or 1,3-alkanediols or -alkenediols, dodecyl thioethers of polyacrylamide, alkoxylated derivatives of fatty acids or of fatty alcohols of lanolin containing at least 50 mol of polar or hydrophilic units constituting the polar head.

13. Pigment according to either of Claims 11 and 12, in which the nonionic amphiphilic compounds correspond to the following formula:
RO-(CH₂CH₂O)ₙ-H
in which R denotes an alkyl, alkenyl or aryl radical containing at least 8 carbon atoms and n is a number ranging from 70 to 200 and more particularly from 100 to 200.

14. Pigment according to any one of Claims 11 to 13, in which the nonionic amphiphilic compounds are present in concentrations ranging from 1 to 40% by weight and preferably from 5 to 20% by weight relative to the total weight of the waxy core and in which the emulsifying surfactant(s)/nonionic amphiphilic compound(s) weight ratio ranges from 1 to 50 and more particularly from 1 to 20.

15. Pigment according to any one of Claims 1 to 14, in which the melanin pigment precursor is chosen from the group consisting of:
(i) indoline compounds of the following formula, and the addition salts thereof with an acid: 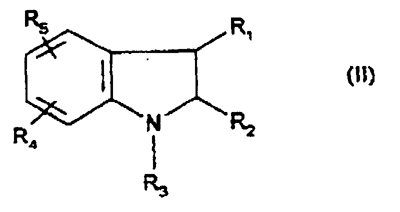 in which:
R₁ and R₃ represent, independently of each other, a hydrogen atom or a C₁-C₄ alkyl group;
R₂ denotes a hydrogen atom, a C₁-C₄ alkyl group, a carboxyl group or a (C₁-C₄)alkoxycarbonyl group;
R₄ denotes a hydrogen atom, a C₁-C₄ alkyl group, a hydroxyl group, a (C₁-C₄) alkoxyamino group, a (C₁-C₁₀) alkylamino group or a halogen atom;
R₅ denotes a hydrogen atom, a C₁-C₄ alkyl group, a hydroxyl group, a C₁-C₄ alkoxy group or an amino group;
at least one of the radicals R₄ or R₅ denoting a hydroxyl, alkoxy or amino group; with the proviso that when R₅ denotes amino then R₄ is other than an alkylamino radical;
R₄ and R₅ may form an alkylenedioxy ring and are in position 5 and 6;
(ii) indole compounds of the following formula, and the addition salts thereof with an acid: 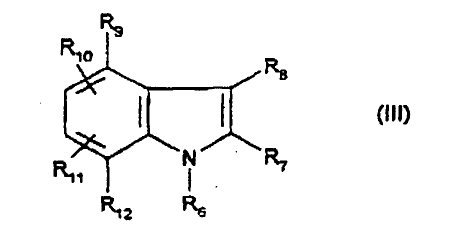 in which:
R₆ and R₈ represent, independently of each other, a hydrogen atom or a C₁-C₄ alkyl group;
R₇ denotes a hydrogen atom, a C₁-C₄ alkyl group, a carboxyl group or a (C₁-C₄)alkoxycarbonyl group;
R₉ and R₁₂ denote, independently of each other, a hydrogen atom, a C₁-C₄ alkyl group, a hydroxyl group, a C₁-C₄ alkoxy group, an amino group, a (C₂-C₄)acyloxy group or a (C₂-C₄)acylamino group;
R₁₀ denotes a hydrogen atom, a C₁-C₄ alkyl group, a hydroxyl group, a C₁-C₄ alkoxy group, an amino group, -a (C₂-C₄)acyloxy group, a (C₂-C₄)acylamino group, a halogen atom or a trimethylsilyloxy group;
R₁₁ denotes a hydrogen atom, a hydroxyl group, a C₁-C₄ alkoxy group, an amino group, a (C₂-C₄)acyloxy group, a (C₂-C₄)acylamino group, a hydroxy(C₂-C₄)alkylamino group or a trimethylsilyloxy group;
R₁₀ and R₁₁ may form, together with the carbon atoms to which they are attached, a methylenedioxy ring optionally substituted with a C₁-C₄ alkyl, a C₁-C₄ alkoxy or a carbonyldioxy ring;
at least one of the groups R₉ to R₁₂ represents a group OZ or NHR and only one of the said groups denotes NHR;
not more than two of the groups R₉ to R₁₂ represent a group OZ, when Z denotes hydrogen and are in position 5 and 6;
at least one of the groups R₉ to R₁₂ denotes hydrogen, in the case where only one of these groups denotes hydrogen, only one of the said groups denotes a group NHR or OZ and the others denote a C₁-C₄ alkyl group;
R denotes a hydrogen atom, a C₂-C₄ acyl group or a C₂-C₄ hydroxyalkyl group;
Z denotes a hydrogen atom, a C₂-C₁₄ acyl group, a C₁-C₄ alkyl group or a trimethylsilyl group.
(iii) mixtures thereof.

16. Pigment according to any one of Claims 1 to 15, in which the melanin pigment precursor is chosen from the group consisting of 5,6-dihydroxyindoline, 6-hydroxyindole, 5,6-dihydroxyindole and N-methyl-5,6-dihydroxyindole and the addition salts thereof with an acid.

17. Pigment according to any one of Claims 1 to 16, in which the melanin pigment precursor(s) is or are used in amounts such that the weight ratio of the wax(es)/dye formed in situ after oxidation is greater than or equal to 3.

18. Pigment according to any one of Claims 1 to 17, in which the size of the spherical particles of composite melanin pigment is less than 1 µm and more preferably less than or equal to 500 nm and more particularly ranges from 20 to 500 nm.

19. Process for the preparation of composite melanin pigment particles as defined according to any one of Claims 1 to 18, **characterized in that** it consists in dispersing and solubilizing, at room temperature, with mild stirring and under an inert atmosphere, a melanin-type precursor as defined according to any one of Claims 1 and 14 to 16 in an aqueous dispersion of spherical wax particles less than 1 µm in size also containing at least one emulsifying surfactant such as those defined above according to any one of Claims 1 to 14, and then in oxidizing the said precursor so as to polymerize it and form an outer layer which envelops each wax particle, and then optionally in drying the aqueous dispersion of composite melanin pigment particles thus obtained so as to obtain a powder.

20. Process according to Claim 19, in which the step of oxidation is carried out below the melting point of the wax or of the wax mixture constituting the wax particles, and preferably at room temperature.

21. Process according to Claim 19 or 20, in which the polymerization step is carried out in aqueous media by autoxidation in air brought about by vigorous stirring of the wax microdispersion which is adjusted to a neutral or alkaline pH.

22. Process according to Claim 19 or 20, in which the polymerization step is carried out in the presence of an oxidizing agent chosen from the group consisting of hydrogen peroxide, peracids and persalts and optionally in the presence of a pH modifier.

23. Process according to Claim 19 or 20, in which the polymerization step is carried out in the presence of an oxidizing agent chosen from the group consisting of alkaline chlorites, silver oxide, ferric chloride, lead oxide, sodium nitrite, rare-earth metal salts, ortho- and para-benzoquinones, ortho- and para-bensoquinone monoimines and diimines, 1,2- and 1,4-naphthoquinones, 1,2- and 1,4-naphthoquinone mono- or diimines, and optionally in the presence of a pH modifier.

24. Process according to Claim 19 or 20, in which the polymerization step is carried out using an iodide ion in the presence of hydrogen peroxide and optionally in the presence of a pH modifier.

25. Process according to Claim 19 or 20, in which the polymerization step is carried out by enzymatic oxidation.

26. Process according to any one of Claims 19 to 25, in which a nonionic amphiphilic compound with a long polar head, as defined according to any one of Claims 11 to 14, is incorporated under hot or cold conditions into the aqueous wax microdispersion before the melanin pigment precursor is dispersed therein.

27. Use of a composite melanin pigment in the form of spherical particles as defined in any one of Claims 1 to 18, in powder form or in the form of an aqueous dispersion, in and for the preparation of a cosmetic or dermatological composition.

28. Cosmetic or dermatological composition comprising at least one composite melanin pigment in the form of spherical particles in powder form or in the form of an aqueous dispersion, as defined in any one of Claims 1 to 18.

29. Composition according to Claim 28, in which the composite melanin pigment is used in powder form or in the form of an aqueous dispersion in a concentration ranging from 0.1 to 35% by weight, and in particular from 0.5 to 20% by weight, relative to the total weight of the composition.

30. Use of a composite melanin pigment in the form of spherical particles, as defined in any one of Claims 1 to 18, in powder form or in the form of an aqueous dispersion, in, and for the preparation of, make-up products for the skin and/or the exoskeleton.

31. Use of a composite melanin pigment in the form of spherical particles, as defined in any one of Claims 1 to 18, in powder form or in the form of an aqueous dispersion, in, and for the preparation of, dye compositions for the hair, in particular for temporarily dyeing or making up the hair.

32. Use of a composite melanin pigment in the form of spherical particles, as defined in any one of Claims 1 to 18, in powder form or in the form of an aqueous dispersion, in, and for the preparation of, compositions for protecting the human epidermis and/or the exoskeleton against the harmful effects of UV rays, in particular so-called antisun products.

33. Use of a composite melanin pigment in the form of spherical particles, as defined in any one of Claims 1 to 18, in powder form or in the form of an aqueous dispersion, in, and for the preparation of, compositions for protecting the skin and/or the exoskeleton against the effects of free radicals induced by atmospheric pollutants and/or by ultraviolet radiation.
